# EUROPEAN PATENT APPLICATION

(11) **EP 4 740 867 A1**
(43) Date of publication of application: **13.05.2026**
(21) Application number: 25208813.3
(22) Date of filing: 15.10.2025
(51) Int. Cl.: A61B 8/08, A61B 5/24, A61B 5/00, A61B 8/00

(54) **SYSTEM FOR GENERATING PHYSIOLOGICAL TIME-SERIES VIA DEEP LEARNING MODEL DETECTION OR PREDICTION**

(30) Priority: 07.11.2024 US 202418940639
(71) Applicant: GE Precision Healthcare LLC, Waukesha, WI 53188 (US)
(72) Inventor: KHAIR, Mohammad, Milwaukee, 53188 (US); MANICKAM, Kalaivani, Solihull, B913UN (GB)
(74) Representative: Kilburn & Strode LLP

(57) **Abstract**

Methods and systems are provided for detecting and predicting uterine activity (UA) from an ultrasound receive signal and/or electrohysterogram (EHG) data, using a neural network model (420) trained using ultrasound time-series data (402) and/or EHG data as input data (404) and time-series tocodynamometer (toco) data as ground truth data (405). After training, the trained UA detection model (430) may be used to detect a timing, duration, and intensity of uterine contractions in new patients with more accuracy than the toco. An output of the trained UA detection model (430) may also be used to train a UA prediction model (470) to predict a next uterine contraction (1210) based on a series of preceding uterine contractions. As a result, the uterine contractions may be detected and/or predicted during electronic fetal monitoring (EFM) without the use of an additional toco device, reducing a cost of the EFM and a number of sensing devices relied on for the EFM.

## Description

### TECHNICAL FIELD

Embodiments of the subject matter disclosed herein relate to detecting or predicting physiological signals of a patient, including fetal heart rate activity and uterine activity in a pregnant mother.

### BACKGROUND

Uterine Activity (UA) corresponding to a mother's uterine contractions may be measured using a tocodynamometer, or toco, which measures a displacement of the mother's abdomen via a belt mounted mechanical pressure transducer. The UA may be represented as time-series data, which may be used to determine a frequency, an amplitude, a duration, and/or a period of the uterine contractions. However, the data generated by the toco may not be as precise as desired, due to a dependence on abdominal position and variable tension on the belt, as well as elevation ambient pressure dynamics that may affect the data.

Clinical ultrasound is an imaging modality that employs ultrasound waves to probe the internal structures of a body of a patient and produce a corresponding physiological signal. For example, an ultrasound probe comprising a plurality of transducer elements emits ultrasonic pulses which reflect or echo, refract, or are absorbed by structures in the body. The ultrasound probe then receives reflected echoes, which are processed into a signal. Transabdominal ultrasound performed on a pregnant mother also captures contractions of the uterine muscle, which is positioned in the path of an abdominal ultrasound beam. However, time-series data generated by a toco may not indicate the frequency and duration of the uterine contractions as accurately as the transabdominal ultrasound. The ultrasound has more sensitivity to tissue compression and is also less positionally dependent when transducer is placed on mother's abdomen, as compared to the Toco.

### SUMMARY

The current disclosure at least partially addresses one or more of the above identified issues via a method, comprising acquiring ultrasound time-series data of an abdomen and/or fetus of a pregnant mother; inputting the ultrasound time-series data into a first trained neural network model; generating a plot of a uterine activity (UA) of the mother from an output of the first trained neural network model; and displaying the plot on a display device.

The above advantages and other advantages, and features of the present description will be readily apparent from the Detailed Description below when taken alone or in connection with the accompanying drawings. It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

Various aspects of this disclosure may be better understood upon reading the following detailed description and upon reference to the drawings in which:
FIG. 1 shows a block diagram of an exemplary ultrasound system;
FIG. 2 shows an exemplary tocodynamometer;
FIG. 3 is a schematic diagram of an exemplary UA detection system used to predict uterine contractions in pregnant mothers;
FIG. 4 is an information flow diagram showing an exemplary system for training and deploying a UA detection model used by the UA detection system;
FIG. 5 is a flowchart illustrating an exemplary method for training the UA detection model;
FIG. 6 is a flowchart illustrating a method for using a UA detection model to predict uterine contractions;
FIG. 7 is a first graph of time-series data showing UA in a pregnant mother, as prior art;
FIG. 8 is a second graph of time-series data showing UA in a pregnant mother, as prior art;
FIG. 9 shows an exemplary array of UA data values extracted from UA time-series data;
FIG. 10 shows an example of UA time-series windows extracted from the array of UA data values;
FIG. 11 is a third graph of time-series data showing UA in a pregnant mother, as prior art;
FIG. 12 is a graph showing a plot of an exemplary output of the UA detection model; and
FIG. 13 is a schematic diagram showing an exemplary neural network architecture of the UA detection model.

### DETAILED DESCRIPTION

Accurately evaluating the well-being of a fetus during labor and delivery may lead to a healthy newborn and mother. Electronic Fetal Monitoring (EFM) is a common obstetrical procedure in the US, and most women undergo EFM during pregnancy, labor, and delivery. The rationale for use of EFM assumes that Fetal Heart Rate (FHR) abnormalities accurately reflect risk of hypoxia (inadequate low oxygenation of the fetus), and that early recognition of hypoxia could induce intervention to improve outcomes for the fetus. EFM may also be used to detect post-partum hemorrhage due to lack of contraction of uterus. EFM is often used to evaluate fetal well-being during the labor process by recording fetal heartbeat and frequency of uterine contractions via two monitors - an ultrasound device and a tocodynamometer (toco), respectively.

During labor, care providers may place the ultrasonic probe over the mother's abdomen above the fetus, once detected, where a consistent fetal heartbeat may be detected. Ultrasonic gel may be applied between the probe and a surface of the mother's abdominal skin. The probe is held to the abdomen with a first elastic belt, wearable patch, or similar apparatus. The toco sensor may be applied to the abdomen above the umbilicus where the fundal height of the uterus is palpated, and may be held to the upper part of the abdomen by a second elastic belt. A heartbeat of the fetus may be detected in the ultrasound data during uterine contractions, while contraction detection via the toco allows monitoring of the progress of labor.

The toco detects the variable presence or absence of tension on the abdomen whether from uterine contraction or maternal movement. However, an accuracy of the toco may be less than desired. The toco may record an amplitude of a uterine contraction with accuracy, but may not record a timing (start and end) or duration of the uterine contraction as accurately. Data acquired via the toco may vary depending on a position and a tension of the second elastic belt. The second elastic belt may be positioned on the mother's abdomen such that a quality of the toco data may be lower than desired, or the belt may slip. If the second elastic belt is too tight, the toco data may show delays and/or poor amplitudes. The toco data may be affected by elevation pressure dynamics, where data may be poor for patients at some elevations. If an amount of abdominal fat is high, such as with an obese mother, detection of the uterine contractions may be difficult or impossible. In some cases, the toco data may not be sufficient to accurately determine a timing, duration, and other characteristics of the uterine contractions. For example, when cervical dilation lags behind the anticipated labor curve, oxytocin is often indicated to induce a more effective contraction pattern. Safe titration of the oxytocin may rely on a unique accurate determination of "Montevideo units" which measure the strength of uterine contractions over 10 minutes. In such cases the toco may not provide sufficiently accurate data, and a more invasive intra-uterine pressure catheter (IUPC) may be placed into the uterus, alongside the fetus, to measure the pressure generated by uterine contractions.

Uterine contractions, or uterine activity (UA) more generally, can also be detected in the unprocessed ultrasound data, meaning, prior to processing the ultrasound data into physiological signals, because transabdominal ultrasound performed on a pregnant mother also captures contractions of the uterine muscle, which is positioned in the path of an abdominal ultrasound beam. During operation of the ultrasound system, transmit signals are typically generated and transmitted to the probe via coaxial cables. A transmit signal refers to a signal comprising a plurality of pulses of high-frequency, high voltage ultrasound energy used to generate an acoustic wave via transducer elements of the probe, which is projected into the mother's abdomen/uterus. The acoustic wave may be reflected back, for example, from anatomical structures of the mother and fetus. The reflected acoustic wave may be received by the transducer elements, which may generate a receive signal. The receive signal may be transmitted via the coaxial cables back to the ultrasound system, which may process the receive signal to generate a physiological signal. Prior to physiological signal generation, an amplitude of the unprocessed receive signal from the probe may be modulated by contractions of the uterine muscle. As a result, the reflected signal generates time-series data from which the contractions can be detected. However, the raw time-series data may be difficult to interpret, where a visualization of the time-series data may not indicate clear beginnings and endings of the contractions. Due to a high variance in the reflected signal, an average amplitude may not be clearly discernable.

Uterine contractions can also be detected and/or measured using an electrohysterogram (EHG). Uterine contractions are the result of the coordinated actions of individual myometrial cells. At the cellular level, the contractions are triggered by a voltage signal called an action potential. During pregnancy, cellular electrical connectivity increases such that the action potential propagates to produce a coordinated contraction involving the entire uterus. The action potential during a uterine contraction can be measured with electrodes placed on the maternal abdomen resulting in a uterine EMG signal. The EHG signal can be processed to produce a signal that is similar to the standard uterine activity signal from the tocodynamometer or IUPC. In addition to contraction frequency and duration information, the EHG may provide an accurate indication of an intensity of a contraction. However, the EHG signal may be noisy due to high frequency electrophysiological muscle activity. It may be difficult to detect features directly within the EHG signal, and relate them to a start, end, duration, and amplitude of a contraction.

To address the problem of accurately recording and predicting uterine contractions, systems and methods are disclosed herein for generating a UA trace from time-series data that may be more accurate than a second trace generated by a toco. Specifically, a UA detection model may be trained to detect uterine contractions in a first set of time-series data, such as ultrasound data, EHG data, or a different type of time-series data collected from a plurality of patients, using a second set of time-series data showing UA, such as toco data, as ground truth data. After training, the trained UA detection model may be used to detect and/or predict uterine contractions in new patients. Because the UA detection model is trained on a large training dataset spanning a wide variety of subjects of different ages, body types, races, etc., the UA detection model may learn to ignore variance across individuals in the first set of time-series data, and as a result, the trained UA detection model may more accurately detect uterine contractions than the toco and other filter-based techniques of the ultrasound system and/or the EHG system.

Using the proposed system, UA signal depth and time can also be predicted from an EHG signal, in a way that eliminates the filtering process and respective delays. The proposed technique can be performed in real time, and can predict UA at a same time as an input signal. Specifically, an output of the trained UA detection model may also be used to train a second, UA prediction model to predict a next uterine contraction from a set of previous uterine contractions. Predicting a next contraction may help to prepare a pregnant mother for pushing and understand the early fetal heart rate deceleration that may occur prior to a contraction, and is indicative of a deoxygenation state of the fetus. As UA prediction (generation) from EHG removes the delay in the UA start detection, the FHR deceleration in the FHR trace can be correlated with a wellbeing of the fetus.

Because the trained UA detection model can be applied to ultrasound data already being recorded to capture a heartbeat of the fetus, a reliance on the toco for contraction detection may be reduced or eliminated. Because ultrasound and/or EHG can detect uterine contractions without pressure sensitivity, the proposed system may enable wearable applications for pre-natal uterine contraction detection, as well as intrapartum UA and post-partum UA, which is important for detection of post-partum hemorrhage. Thus, generative UA from ultrasound, EHG, or a different modality as described herein can replace the use of a toco transducer on the patient, reducing sensing hardware costs, while achieving the equivalent or increased clinical performance.

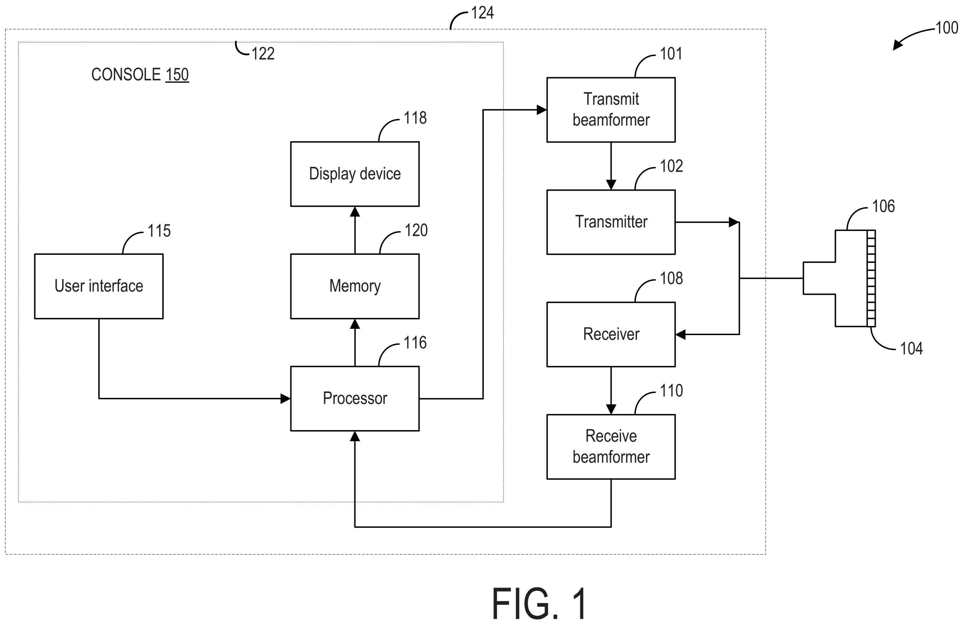

Referring now to FIG. 1, a schematic diagram of an ultrasound system 100 in accordance with an embodiment of the disclosure is shown. The probe 106 includes a transmit beamformer 101 and a transmitter 102 that drives elements (e.g., transducer elements) 104 within a transducer array, herein referred to as probe 106, to emit pulsed ultrasonic signals (referred to herein as transmit pulses) into a body of a patient (not shown). The probe 106 may be a one-dimensional transducer array probe, or may be a two-dimensional matrix transducer array probe. The transducer elements 104 may be comprised of a piezoelectric material, such as lead zirconate titanate (PZT), lead magnesium niobate-lead titanate (PMN-PT), single crystal PMN-PT (PIN-PMN-PT), or a different piezoelectric material. It should be appreciated that the examples provided herein are for illustrative purposes and a different type of piezoelectric material may be used without departing from the scope of this disclosure. When a voltage is applied to a piezoelectric crystal, the crystal physically expands and contracts, emitting an ultrasonic spherical wave. In this way, transducer elements 104 may convert electronic transmit signals into acoustic transmit beams.

After the elements 104 of the probe 106 emit pulsed ultrasonic signals into a body (of a patient), the pulsed ultrasonic signals are back-scattered from structures within an interior of the body, like blood cells or muscular tissue, to produce echoes that return to the elements 104. The echoes are converted into electrical signals, or ultrasound data, by the elements 104 and the electrical signals are received by a receiver 108. The electrical signals representing the received echoes are passed through a receive beamformer 110 that outputs ultrasound data to a processor 116 (e.g., also herein described as controller 116) to be displayed on a display device 118. Additionally, transducer element 104 may produce one or more ultrasonic pulses to form one or more transmit beams in accordance with the received echoes. The terms "scan" or "scanning" may also be used in this disclosure to refer to acquiring data through the process of transmitting and receiving ultrasonic signals.

Components of the ultrasound system 100 shown in FIG. 1 may be included in a console 150, or may be included in the probe 106, where the probe 106 may be electronically coupled to the console 150 via a cable. In some embodiments, the probe 106 may contain electronic circuitry to do all or part of the transmit beamforming and/or the receive beamforming. For example, all or part of the transmit beamformer 101, the transmitter 102, the receiver 108, and the receive beamformer 110 may be situated within the probe 106, where the console 150 may include the components depicted within dotted lines 122. In other embodiments, the console 150 may include the transmit beamformer 101, the transmitter 102, the receiver 108, and the receive beamformer 110, where the console 150 may include the components depicted within dashed lines 124. It should be appreciated that the ultrasound system 100 depicted in FIG. 1 is for illustrative purposes, and in other embodiments the ultrasound system 100 may include a greater or lesser number of components located either in the probe 106 or the console 150.

The console 150 may include a user interface 115, which may be used to control operation of the ultrasound system 100. The user interface 115 may include one or more of the following: a rotary element, a mouse, a keyboard, a trackball, hard keys linked to specific actions, soft keys that may be configured to control different functions, and/or a graphical user interface displayed on the display device 118.

The processor 116 may control the transmit beamformer 101. The processor 116 is in electronic communication (e.g., communicatively connected) with the probe 106. The term "electronic communication" may be defined to include both wired and wireless communications, although, for purposes of this disclosure, the probe 106 may be coupled to the console 150 via a first set of wires used to send a transmit signal to transmit beamformer 101, and a second set of wires used to receive a receive signal from receive beamformer 110 (e.g., the cable). The processor 116 may control the probe 106 to acquire data according to instructions stored on a memory 120.

As discussed herein, memory includes any non-transient computer readable medium in which programming instructions are stored. For the purposes of this disclosure, the term tangible computer readable medium is expressly defined to include any type of computer readable storage. The example methods and systems may be implemented using coded instruction (e.g., computer readable instructions) stored on a non-transient computer readable medium such as a flash memory, a read-only memory (ROM), a random-access memory (RAM), a cache, or any other storage media in which information is stored for any duration (e.g. for extended period time periods, permanently, brief instances, for temporarily buffering, and/or for caching of the information). Computer memory of computer readable storage mediums as referenced herein may include volatile and non-volatile or removable and non-removable media for a storage of electronic-formatted information such as computer readable program instructions or modules of computer readable program instructions, data, etc. that may be stand-alone or as part of a computing device. Examples of computer memory may include any other medium which can be used to store the desired electronic format of information and which can be accessed by the processor or processors or at least a portion of a computing device.

The processor 116 and the transmit beamformer 101 control which of the elements 104 are active and the shape of a beam emitted from the probe 106. The processor 116 is in electronic communication with the display device 118, and the processor 116 may process the data (e.g., ultrasound data) into physiological signals for display on the display device 118. The processor 116 may include a central processor (CPU) and/or graphic processing units (GPU), according to an embodiment. According to other embodiments, the processor 116 or transmit beamformer 101 may include other electronic components capable of carrying out processing functions, such as a microprocessor, a microcontroller, or a field-programmable gate array (FPGA). According to other embodiments, the processor 116 may include multiple electronic components capable of carrying out processing functions. For example, the processor 116 may include two or more electronic components selected from a list of electronic components including: a central processor, a digital signal processor, an FPGA, and a graphic board. According to another embodiment, the processor 116 may also include a complex demodulator (not shown) that demodulates the received signals and generates raw data. In another embodiment, the demodulation can be carried out earlier in the processing chain (e.g. receive beamformer 110).

The processor 116 may be adapted to perform one or more processing operations according to a plurality of selectable ultrasound modalities on the data. In one example, the data may be processed in real-time during a scanning session as the echo signals are received by receiver 108 and transmitted to processor 116. For the purposes of this disclosure, the term "real-time" is defined to include a procedure that is performed without any intentional delay. For example, an embodiment may acquire physiological signals at a real-time rate of 4-20 samples/sec. Some embodiments of the invention may include multiple processors (not shown) to handle the processing tasks that are handled by processor 116 according to the exemplary embodiment described hereinabove. For example, a first processor may be utilized to demodulate and decimate the received signals while a second processor may be used to further process the data, for example by augmenting the data as described further herein, prior to displaying a physiological signal. It should be appreciated that other embodiments may use a different arrangement of processors.

The ultrasound system 100 may continuously acquire data at a sample-rate of, for example, 4 Hz to 30 Hz (e.g., 10 to 30 frames per second). Physiological signals may be generated from the data may be refreshed at a similar sample-rate on display device 118. Other embodiments may acquire and display data at different rates. For example, some embodiments may acquire data at a sample-rate of less than 4 Hz or greater than 30 Hz depending on the size of the frame and the intended application. A memory 120 is included for storing processed frames of acquired data. In an exemplary embodiment, the memory 120 is of sufficient capacity to store at least several seconds' worth of frames of ultrasound data. The frames of data are stored in a manner to facilitate retrieval thereof according to its order or time of acquisition. The memory 120 may comprise any known data storage medium.

In various embodiments of the present disclosure, data may be processed in different mode-related modules by the processor 116 (e.g., B-mode, Color Doppler, M-mode, Color M-mode, spectral Doppler, Elastography, TVI, strain, strain rate, and the like) to form 2D or 3D data. For example, one or more modules may generate B-mode, color Doppler, M-mode, color M-mode, spectral Doppler, Elastography, TVI, strain, strain rate, and combinations thereof, and the like. As one example, the one or more modules may process color Doppler data, which may include traditional color flow Doppler, power Doppler, HD flow, and the like. The received ultrasound signals are stored in memory and may include timing information indicating a time at which the physiological time-series signal samples were stored in memory. The modules may include, for example, a scan conversion module to perform scan conversion operations to convert the acquired physiological signals from beam space coordinates to display space coordinates. A video processor module may be provided that reads the acquired physiological signals from a memory and displays physiological signals in real time while a procedure (e.g., ultrasound imaging) is being performed on a patient. The video processor module may include a separate physiological signals memory, and the ultrasound physiological signals may be written to the physiological signals memory in order to be read and displayed by display device 118.

After performing an ultrasound scan, a block of data comprising scan lines and their samples is generated. After back-end filters are applied, a process known as scan conversion is performed to transform the data block into a displayable physiological signal with additional scan information such as depths, angles of each scan line, and so on. During scan conversion, an interpolation technique is applied to fill missing holes (e.g., samples) in the resulting physiological signals. These missing samples occur because each element of the transducers should typically cover many samples in the resulting signal.

In embodiments where the transmit beamformer 101 is included in the console 150 and not in the probe 106, the ultrasound system 100 may be configured to transmit a signal from transmit beamformer 101 to the probe 106 for operating the probe 106. In embodiments where the transmit beamformer 101 is included in the probe 106 and not in the console 150, the ultrasound system 100 may be configured to transmit beam setup data from the processor 116 (e.g., and/or from an FPGA of the processor 116) to transmit beamformer 101 for operating probe 106. The beam setup data may be transmitted as low voltage digital signals, as opposed to the high voltage transmit signal. A dedicated set of wires may be used for such purposes. However, a number of wires in the dedicated set of wires may be small (e.g., four), and an amount of beam setup data transmitted from the processor 116 may be large, which may cause delays in operating probe 106 due to slow data transfer rates.

In addition to generating physiological signals, an amplitude of the reflected receive signal may also be recorded over predefined increments of time to generate time-series data. In particular, during transabdominal ultrasound procedures performed on pregnant mothers as part of electronic fetal monitoring, the time-series data may be used to detect a heartbeat of a fetus of a pregnant mother, or to monitor uterine activity before, during, or after labor. As described in greater detail below, a UA detection system may be advantageously applied to the time-series data that may detect and/or predict uterine contractions of the mother during labor.

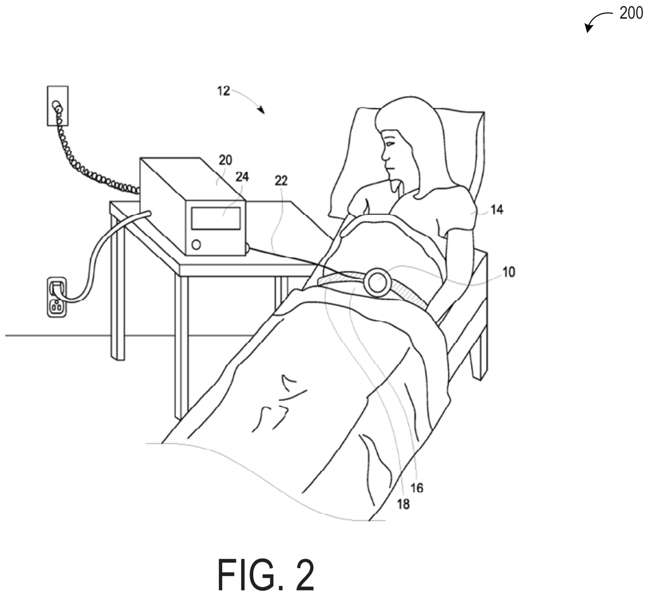

As mentioned above, conventionally, the uterine activity of the pregnant patient is typically monitored using a toco. The toco uses pressure or displacement to detect uterine contractions. In one embodiment of a toco, a pressure transducer, such as a depressible surface, is affixed to the abdomen of the pregnant patient such that the pressure of the abdomen may be detected, and the contractions monitored. Alternatively, the toco may utilize a strain gauge disposed between one or more points affixed to locations on the patient's abdomen, such that the expansion and the contraction of the patient's abdomen may be detected. An advantage of a toco is that the detected pressure, displacement, or strain not only provides an indication of contraction onset, but relative strength of the contractions. An exemplary toco is shown in FIG. 2.

FIG. 2 is an environmental view of an exemplary embodiment of a maternal and fetal monitoring system 12 that includes use of a toco transducer 10, also referred to herein as toco 10, which may be used for simultaneously monitoring the health of a maternal patient and fetal patient. Toco 10 is exemplarily secured to the abdomen 16 of the maternal patient 14, for example, by way of an elastomeric strap 18. However, it will be recognized that in other embodiments, a biocompatible adhesive may be used to secure the toco 10 to the patient's abdomen 16.

The toco 10 may be communicatively connected to a monitoring device 20 by a communicative connection 22, which may be a wired or a wireless communicative connection. Depending upon the configuration of the maternal and fetal monitoring system and the data transmitted between the toco 10 and the monitoring device 20, some or all of the data processing of the physiological information acquired the transducer may be performed locally at the toco 10. A controller located within the toco 10 may receive the acquired physiological data and process such physiological data in the manners as described herein and the calculated parameters of fetal heart rate (HR), maternal HR, UA or others as described herein may be communicated across the communicative connection 22 to the monitoring device 20 exemplarily for visual presentation on a graphical display 24 and/or electronic storage of this information on a data network of the hospital or medical facility and exemplarily in a electronic medical record (EMR) of the maternal patient.

In other embodiments, the toco 10 may perform more limited signal processing on the acquired physiological data and provide this physiological data across the communicative connection 22 to a monitoring device 20 which applies the signal processing actions and techniques as described herein to calculate the parameters of fetal HR, maternal HR, UA, and others.

The toco 10 includes an ultrasound interface constructed of an acoustically conductive material. A plurality of ultrasound crystals may be located within a housing of the toco 10 behind the ultrasound interface 28. In use, an acoustic enhancing substance which may be a liquid, paste, gel, or solid material may be interspersed between the ultrasound interface and the abdomen 16 of the maternal patient. The toco 10 further includes a plurality of electrodes arranged in the housing on a patient side of the toco 10 surrounding the ultrasound interface. The electrodes are configured to interface with the skin of the maternal patient to acquire electric biopotentials including biopotential components from the maternal heart, a fetal heart, and other biopotential sources, including electromyographical (EMG) signals from the maternal and/or fetal patient.

In a non-limiting exemplary embodiment, the electrodes may be constructed from silver and/or silver chloride which may be formed as a foil or a conductive ink for construction of printed electronics. Additionally, at least one of the electrodes may include a plurality of biocompatible conductive needles wherein each needle has a length between 10 micrometers and 200 micrometers. Such needles have been found to assist in providing an improved electric connection, for example by providing micro abrasion of the patient's skin through the stratum corneum to more conductive layers of skin below.

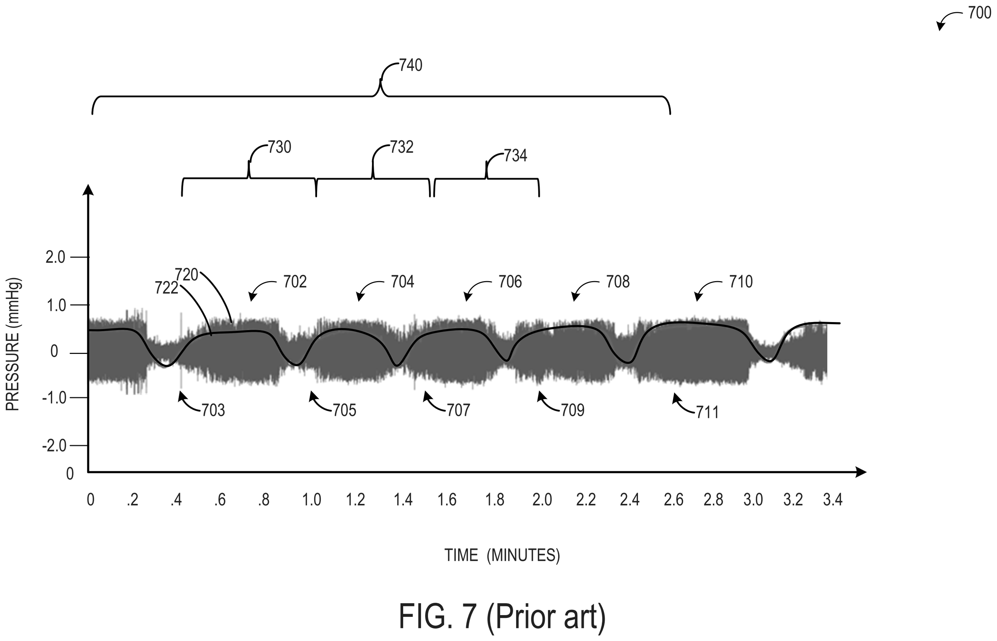

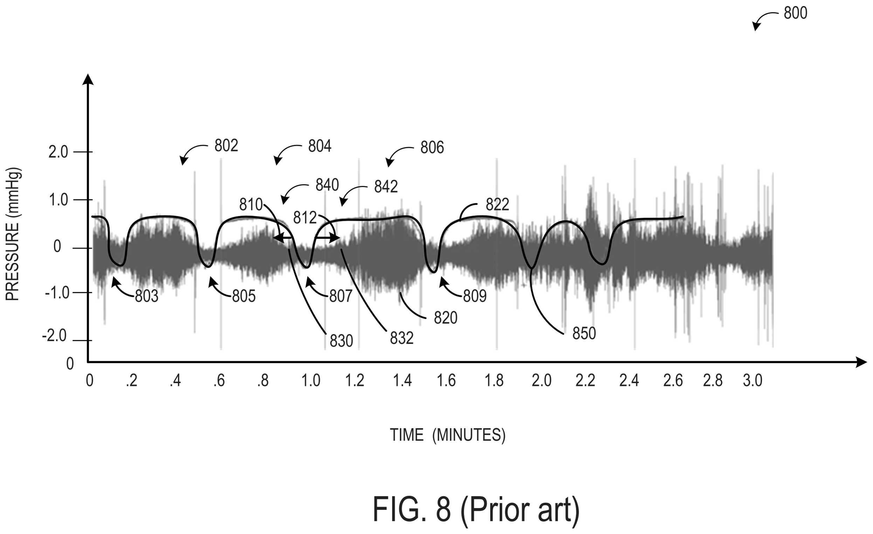

FIGS. 7 and 8 provide an overview of how a UA detection system may be used in conjunction with ultrasound system 100 to detect and/or predict characteristics of uterine contractions of a mother during labor, including a duration, timing, amplitude, and intensity of the contractions. The figures show a concurrence of a first set of time-series data generated by a reflected ultrasound signal and a second set of time-series data generated by a toco, which may be exploited by the UA detection system to increase an accuracy of contraction detection.

Referring to FIG. 7, a time-series graph 700 is shown of ultrasound data indicating a UA of a patient as a transabdominal ultrasound scan is being performed using an ultrasound system, such as ultrasound system 100 of FIG. 1. The UA is concurrently detected by a toco, such as toco 200 of FIG. 2, and time-series data generated by the toco is superimposed on the ultrasound data. Pressure measurements recorded in reflected raw ultrasound data acquired using an ultrasound probe and the toco are indicated on a vertical axis of time-series graph 700 in mmHg, and time is indicated on a horizontal axis of time-series graph 700 in minutes. Note that FIG. 7 shows inverted UA activity.

Time-series graph 700 shows various uterine contractions, which are represented in an ultrasound plot 720 and a superimposed toco plot 722. For example, ultrasound plot 720 may be generated by an ultrasound probe placed or worn at a first location on an abdomen of the patient, and toco plot 722 may be generated by sensors arranged on an elastic belt worn by the patient at a second location on the abdomen.

Each uterine contraction of the various uterine contractions begins and ends with a sharp reduction in pressure as recorded by the toco. A first uterine contraction 702 begins with a first pressure reduction at a point 703 and ends with a second pressure reduction at a point 705; a second uterine contraction 704 begins with the second pressure reduction at point 705 and ends with a third pressure reduction at a point 707; a third uterine contraction 706 begins with the third pressure reduction at point 707 and ends with a fourth pressure reduction at a point 709; a fourth uterine contraction 708 begins with the fourth pressure reduction at point 709 and ends with a fifth pressure reduction at a point 711; and so on. Each pressure reduction is characterized by a relaxation of a uterine muscle of the patient in between respective uterine contractions. The reductions in pressure are also visible in ultrasound plot 720.

Each uterine contraction may have a slightly different duration, due to normal variability in the UA of the patient. For example, first uterine contraction 702 may have a first duration 730; second uterine contraction 704 may have a second duration 732, where second duration 732 is shorter or longer than first duration 730; and third uterine contraction 706 may have a third duration 734, where third duration 734 may be shorter or longer than either or both of first duration 730 and second duration 732. For example, in FIG. 7, second uterine contraction 704 and third uterine contraction 706 have a slightly shorter duration than first uterine contraction 702.

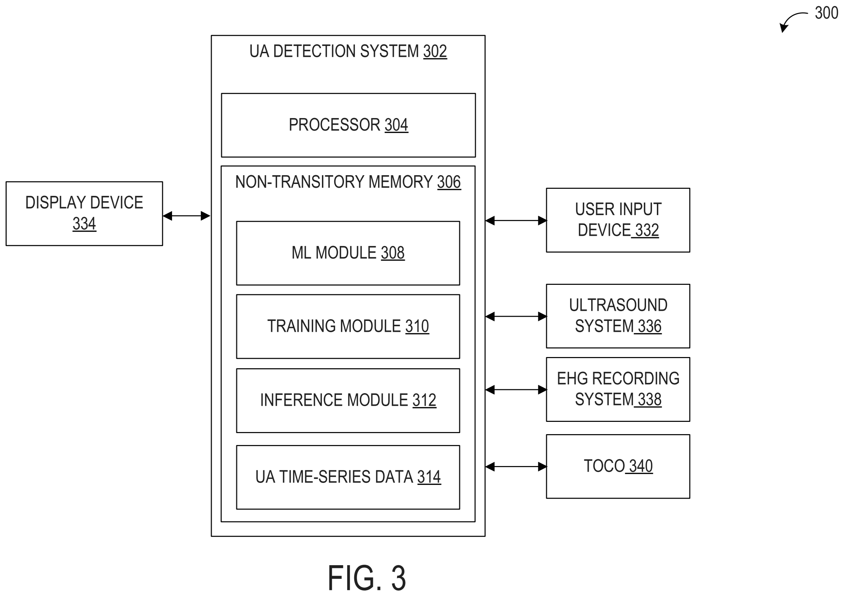

In order to detect the uterine contractions based on the ultrasound data, a moving window 740 of time-series UA data may be analyzed by a UA detection system, such as the UA detection system described below in reference to FIG. 3. Moving window 740 may comprise a plurality of uterine contractions. In FIG. 7, moving window 740 comprises first uterine contraction 702, second uterine contraction 704, third uterine contraction 706, and a fourth uterine contraction 708. Based on the time-series UA data included in moving window 740, a subsequent contraction 710 may be predicted by the UA detection system, in some examples.

FIG. 8 shows a second time-series graph 800 of a UA of a patient as a transabdominal ultrasound scan is being performed using an ultrasound system, similar to FIG. 7. Time-series graph 800 shows various uterine contractions, which are represented in an ultrasound plot 820 and a toco plot 822. Note that FIG. 8 shows inverted UA activity.

According to the toco data, first uterine contraction 802 begins at a point 803 and ends at a point 805; a second uterine contraction 804 begins at point 805 and ends at a point 807; and a third uterine contraction 806 begins at point 807 and ends at a point 809. However, unlike first time-series graph 700, the data of ultrasound plot 820 and toco plot 822 do not align neatly. For example, the data of ultrasound plot 820 shows an end of second uterine contraction 804 at a point 830 rather than at point 807 indicated by toco plot 822. Thus, toco plot 822 would more accurately reflect second uterine contraction 804 if a portion 840 of toco plot 822 were adjusted earlier in time, as indicated by an arrow 810. On the other hand, the data of ultrasound plot 820 shows a beginning of third uterine contraction 806 at a point 832, rather than at point 807 indicated by toco plot 822. Thus, toco plot 822 would more accurately reflect third uterine contraction 806 if a portion 842 of toco plot 822 were adjusted later in time, as indicated by an arrow 812.

Because the ultrasound data underlying ultrasound plot 820 is not pressure sensitive, while the toco sensors are arranged on a belt which may not be at an ideal position, are highly position sensitive, and/or not have an ideal tightness, the ultrasound data may more precisely capture the UA. However, the UA may not be as easy to visualize in ultrasound plot 820 as in toco plot 822. For example, toco plot 822 shows an end of one uterine contraction and a beginning of a next contraction at a point 850, while the transition is not discernable in ultrasound plot 820 at point 850. In other words, in general, ultrasound data may generally indicate the UA more precisely than the toco data, but may lack the clarity of the toco plot.

To address the defects of both of the ultrasound data and the toco data, a UA detection system is proposed that relies on a UA detection model trained using ultrasound data as input data, and toco data as ground truth data. By training the UA detection model on a large population of different subjects, the UA detection model can learn to detect uterine contractions based on concurrences between the ultrasound data and the toco data across the population of different subjects, where an influence of sporadic differences between the ultrasound data and the toco data may be reduced. As a result of training the UI detection model in this manner, predicted UA from ultrasound enables higher sensitivity for the start of and ending of the uterine contractions more accurately than the toco pressure transducers. It can also be applied as a wearable patch that does not rely on a belt wrapped on the belly with sufficient pressure to detect uterine contractions, as in the toco application.

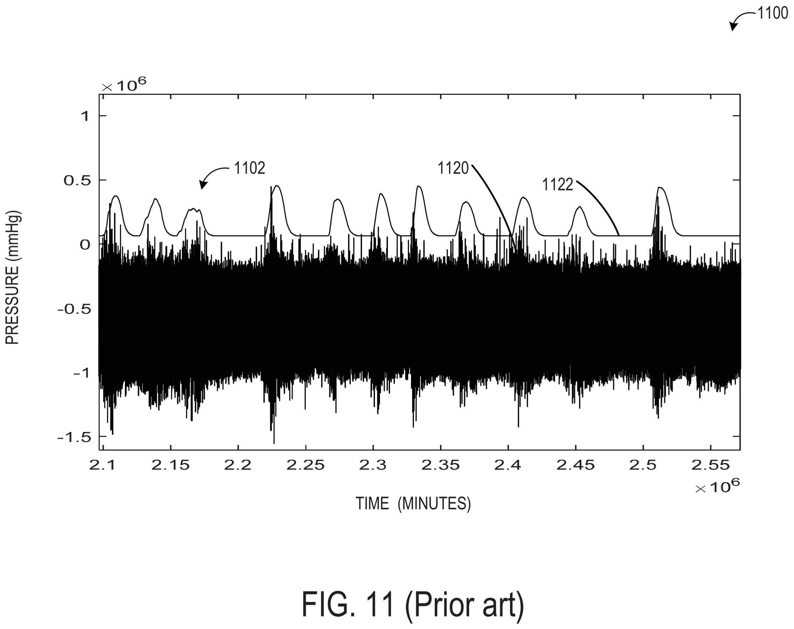

The proposed UA detection system may also be trained using EHG data as input data. FIG. 11 shows an exemplary time-series graph 1100 of EHG data indicating a UA of a patient. As in FIGS. 7 and 8, the UA is concurrently detected by a toco, such as toco 200 of FIG. 2, and time-series data generated by the toco is superimposed on the EHG data. Pressure measurements recorded by the EHG and the toco are indicated on a vertical axis of time-series graph 1100 in mmHg, and time is indicated on a horizontal axis of time-series graph 1100 in minutes. Time-series graph 1100 shows various uterine contractions 1102, which are represented in an EHG plot 1120 and a superimposed toco plot 1122. Each uterine contraction 1102 can be detected in the EHG data as a series of rapid increases or decreases (e.g., spikes) in pressure that occur at regular intervals. The superimposed toco plot 1122 also shows a timing and duration of the uterine contractions 1102, which coincide with the spikes in the EHG data. As with FIG. 8, the uterine contractions are of varying durations and occur at slightly different intervals. The EHG data captures the UA more precisely, but does not clearly show beginnings and endings each contraction. Toco plot 1122 shows the beginnings and endings more clearly, but they may not be perfectly aligned with the UA shown in the EHG data.

The UA detection model may be additionally or alternatively trained using EHG as an input signal and using UA data from either an intrauterine pressure transducer (IUP) or an abdominal toco transducer for ground truth data. Training the UA detection model on the EHG data may result in a more accurate detection of uterine contraction intensity variations, as well as timing including the start and stop of the contraction due to its increased sensitivity.

Referring now to FIG. 3, a block diagram 300 shows a UA detection system 302, in accordance with an embodiment. In some embodiments, UA detection system 302 may be incorporated into an ultrasound system, such as ultrasound system 100 of FIG. 1. In some embodiments, at least a portion of UA detection system 302 is disposed at a device (e.g., edge device, server, etc.) communicably coupled to ultrasound system 100 via wired and/or wireless connections. In some embodiments, UA detection system 302 may be operably/communicatively coupled to a user input device 332 and a display device 334. For example, user input device 332 and display device 334 may be included within operator console 150 of ultrasound system 100, at least in some examples.

UA detection system 302 includes a processor 304 configured to execute machine readable instructions stored in non-transitory memory 306. Processor 304 may be single core or multi-core, and the programs executed thereon may be configured for parallel or distributed processing. In some embodiments, processor 304 may optionally include individual components that are distributed throughout two or more devices, which may be remotely located and/or configured for coordinated processing. In some embodiments, one or more aspects of processor 304 may be virtualized and executed by remotely-accessible networked computing devices configured in a cloud computing configuration.

Non-transitory memory 306 may store a machine learning (ML) module 308, a training module 310, an inference module 312, and UA time-series data 314. Machine Learning (ML) module 308 may include one or more ML models, and instructions for implementing the one or more ML models to predict UA (e.g., uterine contractions) of a patient of the ultrasound system from physiological time-series data of the patient, such as ultrasound time-series data and/or maternal abdominal electrohysterogram (EHG) data, as described in greater detail below. ML module 308 may include models of various types, including trained and/or untrained neural networks such as Convolutional Neural Networks (CNNs), statistical models, or other models, and may further include various data, or metadata pertaining to the one or more models stored therein. In particular, ML module 308 may include a UA detection model for predicting a UA of the patient based on UA data continuously collected during a preceding time-series window.

UA detection system 302 may be operably/communicatively coupled to an ultrasound system 336, an EHG recording system 338, and/or a tocodynamometer (toco) 340 which may provide ultrasound, EHG, and toco data, respectively, used for training the UA detection model and/or for predicting a UA of a patient of the ultrasound system using a trained UA detection model.

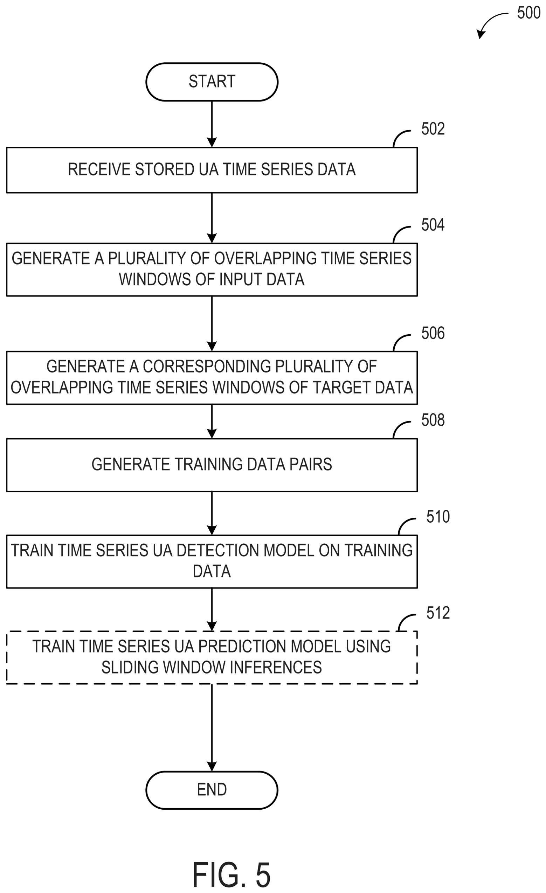

Non-transitory memory 306 may further store a training module 310, which may comprise instructions for training one or more of the models stored in ML module 308. In particular, training module 310 may include instructions that, when executed by processor 304, cause UA detection system 302 to conduct one or more of the steps of method 500 for training a UA detection model, described below in reference to FIG. 5. In some embodiments, training module 310 may include instructions for implementing one or more gradient descent algorithms, applying one or more loss functions, and/or training routines, for use in adjusting parameters of one or more ML models of ML module 308. Training module 310 may include training datasets for the one or more models of ML module 308.

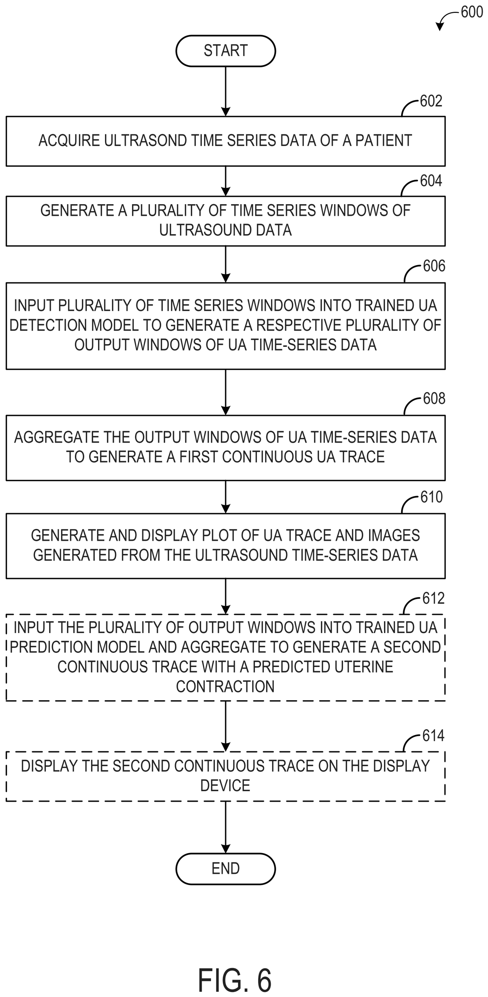

Non-transitory memory 306 also stores an inference module 312. Inference module 312 may include instructions for deploying a trained UA detection model to predict uterine contractions of a patient from physiological time-series data, as described below with respect to FIG. 6. In particular, inference module 312 may include instructions that, when executed by processor 304, cause UA detection system 302 to conduct one or more of the steps of method 600.

Non-transitory memory 306 further stores UA time-series data 314. UA time-series data 314 may include physiological time-series data collected via various medical devices or systems, where the physiological time-series data includes indications of uterine contractions of a pregnant mother. For example, UA time-series data 314 may include transabdominal ultrasound time-series data acquired via ultrasound system 336; EHG data acquired via EHG recording system 338, and/or toco data acquired via toco 340. UA time-series data 314 may include, for example, UA data collected from a plurality of patients and stored in a database. UA time-series data 314 may be collected from one or more patients during a moving time-series window, which may be used in one or more training sets for training the UA detection model of ML module 308, or for predicting a UA of a patient using a trained UA detection model.

User input device 332 may comprise one or more of a touchscreen, a keyboard, a mouse, a trackpad, or other device configured to enable a user to interact with and manipulate data within UA detection system 302. In one example, user input device 332 may enable a user to collect UA data from a patient of the ultrasound system, and/or select UA data from one or more patients of the ultrasound system for training purposes.

Display device 334 may include one or more display devices utilizing virtually any type of technology. In some embodiments, display device 334 may comprise a computer monitor. Display device 334 may be combined with processor 304, non-transitory memory 306, and/or user input device 332 in a shared enclosure, or may be peripheral display devices and may comprise a monitor, touchscreen, projector, or other display device known in the art.

It should be understood that UA detection system 302 shown in FIG. 3 is for illustration, not for limitation. Another appropriate UA detection system may include more, fewer, or different components.

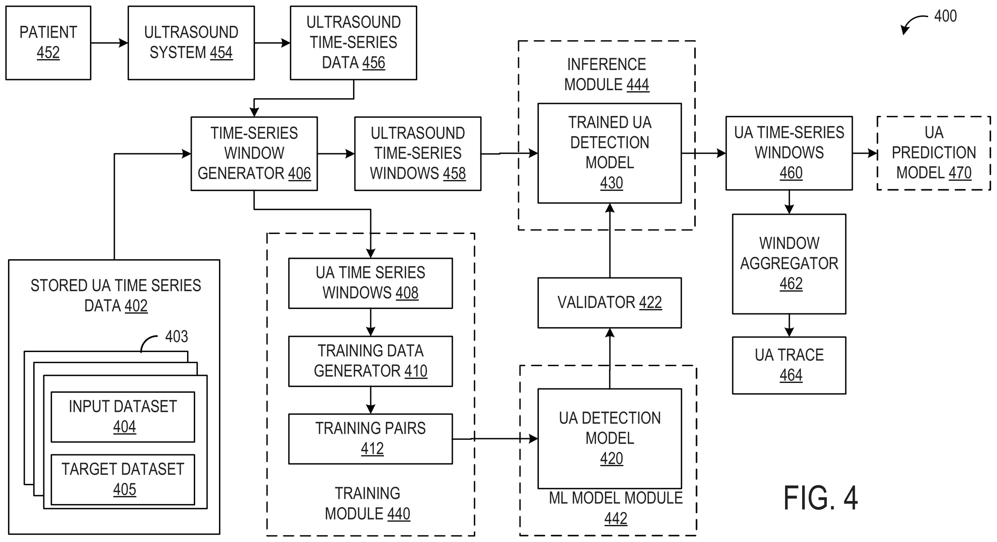

Referring to FIG. 4, an exemplary UA detection model training system 400 is shown, which may be used to train a UA detection model 420. UA detection model 420 may be trained to detect and/or predict uterine contractions of a patient of an ultrasound system during an ultrasound scan. More specifically, UA detection model may be trained to output a time-series signal that represents the detected or predicted uterine contractions.

UA detection model 420 may be used by a UA detection system, such as UA detection system 302 of FIG. 3, in conjunction with an ultrasound system, such as ultrasound system 100 of FIG. 1. UA detection model 420 may be stored within a ML model module 442 of the UA detection system. ML model module 442 may be a non-limiting example of ML module 308 of UA detection system 302 of FIG. 3.

UA detection model 420 may be trained on stored UA time-series data 402 collected from a plurality of human subjects. The plurality of human subjects may include pregnant mothers drawn from a wide population to generate a diverse set of UA data. For example, the plurality of human subjects may include mothers of different ages, races, body types, and sizes. The plurality of human subjects may also include healthy subjects and subjects suffering from one or more health conditions. In various embodiments, stored UA time-series data 402 may be time-series data represented as a continuous stream of recorded physiological patient data over a plurality of predefined increments of time. In various embodiments, stored UA time-series data 402 may be collected from a variety of patients of a healthcare system.

UA time-series data 402 may include different types of physiological time-series data acquired from a plurality of patients (e.g., subjects) 403. The different types of physiological time-series data may be collected from each patient 403 at a same time. UA time-series data 402 includes at least an input dataset 404 having a first type and a target dataset 405 having a second type. That is, for each patient 403, input dataset 404 may include physiological time-series data of the first type that will be inputted into UA detection model 420 during training of UA detection model 420, and target dataset 405 may include physiological time-series data of the second type, collected at a same time, that will be used as ground truth data for training UA detection model 420. For example, input dataset 404 may include transabdominal ultrasound data acquired from a first patient 403 during an ultrasound scan, and target dataset 405 may include toco data acquired from the first patient using a toco worn by the first patient at a time of the ultrasound scan. As a second example, input dataset 404 includes EHG data acquired from a second patient 403, and target dataset 405 includes toco data acquired from the second patient 403 using a toco worn by the second patient.

UA detection model training system 400 may include a time-series window generator 406, which may extract, for a plurality of patients 403 of stored UA time-series data 402, portions of input dataset 404 and target dataset 405 corresponding to discrete, time-series windows 408. For example, a time-series window 408 may comprise five seconds of time-series data from input dataset 404, and five seconds of time-series data from target dataset 405 collected at a same time from a same patient 403, or 10 seconds of time-series data from input dataset 404, and 10 seconds of time-series data from target dataset 405 collected at a same time from a same patient 403, or a different amount of time. The time-series windows 408 may be overlapping, as described in greater detail below.

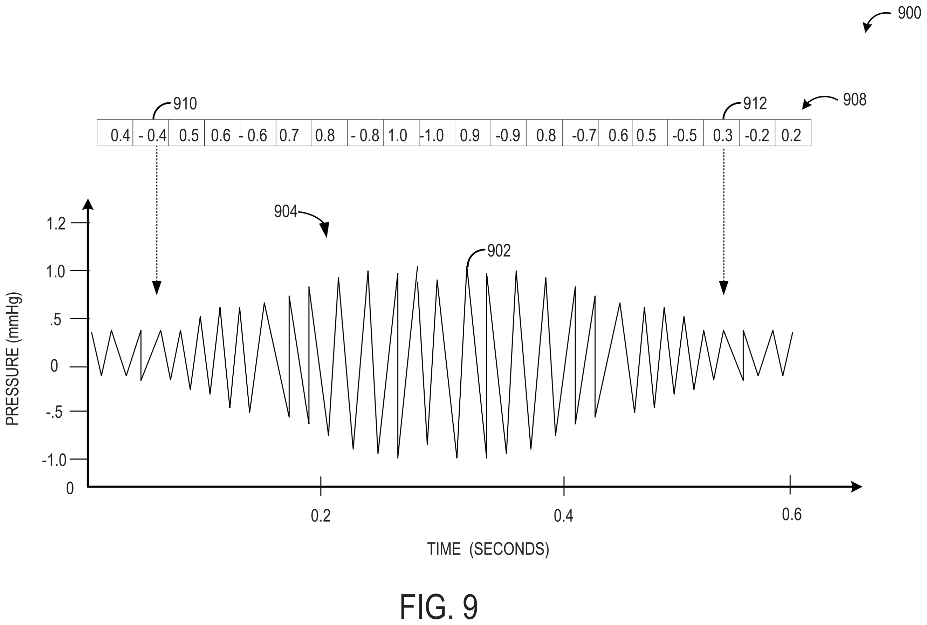

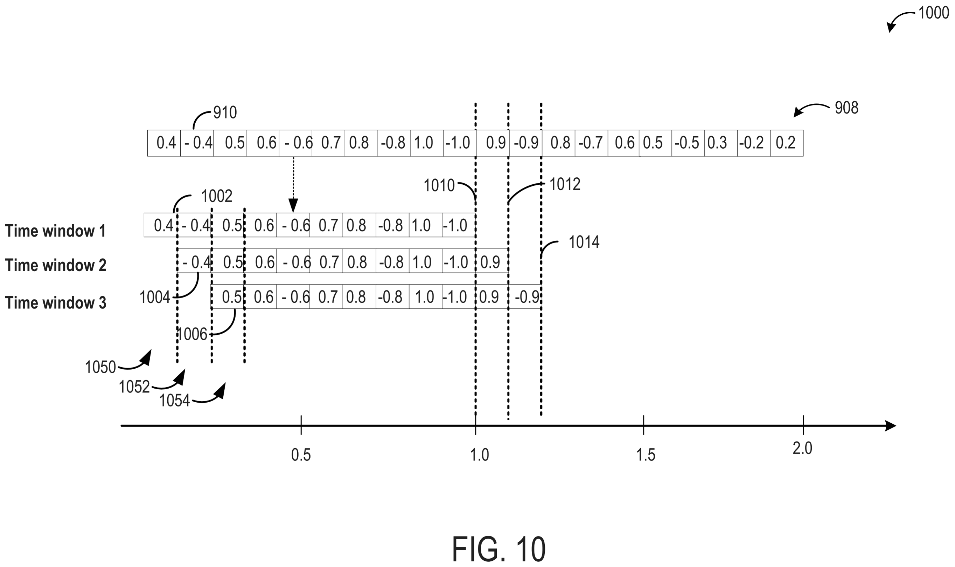

During training of UA detection model 420, time-series window generator 406 may extract time-series windows 408 from both of input dataset 404 and target dataset 405 of one or more subjects included in stored UA time-series data 402. That is, a first plurality of time-series windows 408 may be extracted from a first patient of UA time-series data; a second plurality of time-series windows 408 may be extracted from a second patient of UA time-series data; a third plurality of time-series windows 408 may be extracted from a third patient of UA time-series data; and so on. Additionally, UA time-series windows 408 may be overlapping time-series windows, as described below in reference to FIGS. 9 and 10. The first plurality of time-series windows 408, the second plurality of time-series windows 408, and the third plurality of time-series windows 408 may be used to train UA detection model 420. In this way, UA detection model 420 may be trained on time-series UA data collected from a variety of subjects.

During extraction of the UA time-series windows 408 from the stored UA time-series data 402, portions of stored UA time-series data 402 may be stored in a data array in a buffer in a memory of the UA detection system (e.g., non-transitory memory 306 of UA detection system 302), and each UA time-series windows 408 may be selected from the data array based on an initial time reference, as described below in reference to FIGS. 5, 9, and 10.

UA detection model training system 400 may include a training data generator 410, which may generate a plurality of training pairs 412. Training data generator 410 may be stored in a training module 440 of the UA detection system, such as training module 310 of UA detection system 302 of FIG. 3. Each training pair 412 may include a first time-series window 408 of input dataset 404 of a subject as input data, and a second, corresponding time-series window 408 of target dataset 405 of the subject as ground truth data. Thus, during training of UA detection model 420, UA detection model 420 may learn to map the first time-series window 408 to the second time-series window 408.

For example, referring back to FIG. 7, an exemplary training pair could include a first time-series window 408 comprising 3.4 seconds of ultrasound time-series data represented by plot 720 as input data. A second time-series window 408 comprising 3.4 seconds of simultaneous toco data represented by plot 722 may be used as the ground truth data. During training, UA detection model 420 may learn to output a trace similar to toco plot 722, but drawn to contours of plot 720. The trace may more accurately represent characteristics of the uterine contractions including timing, duration, and intensity than plot 722.

A first portion of training pairs 412 may be assigned to a training dataset, a second portion of training pairs 412 may be assigned to a validation dataset, and a third portion of training pairs 412 may be assigned to a test dataset. In an embodiment, training pairs 412 may be assigned to either the training dataset, the validation dataset, or the test dataset randomly in a pre-established proportion. For example, training pairs 412 may be assigned to either the training dataset or the test dataset randomly such that 90% of the training pairs are assigned to the training dataset, and 10% of the training pairs are assigned to the test dataset and the validation dataset. It should be appreciated that the examples provided herein are for illustrative purposes, and the training pairs may be assigned to the training, validation, and test datasets via a different procedure and/or in a different proportion without departing from the scope of this disclosure.

UA detection model 420 may then be trained on training pairs 412 to learn to detect uterine contractions. UA detection model 420 may be stored in an ML model module 442 of the UA detection system, such as ML module 308 of UA detection system 302 of FIG. 3. An example method for training UA detection model 420 is described in further detail below with respect to FIG. 5.

UA detection model training system 400 may include a validator 422 that validates the performance of the UA detection model 420 using training pairs of the validation dataset and the test dataset. Validator 422 may use the validation dataset to prevent overfitting, and may output an assessment of a performance of the trained or partially trained UA detection model 420 on the test dataset of training pairs. Training may be completed when UA detection model 420 achieves a minimum error rate. After UA detection model 420 is validated, UA detection model 420 may be fully trained. Trained UA detection model 430 may be deployed in an inference module 444 of the UA detection system (e.g., inference module 312 of FIG. 3).

After training has been finished, trained UA detection model 430 may be used by the UA detection system to detect uterine contractions of a patient 452 based on data acquired during an ultrasound scan, via an ultrasound system 454 (e.g., ultrasound system 100 of FIG. 1). UA time-series data 456 of patient 452 may be acquired by ultrasound system 454. From UA time-series data 456, time-series window generator 406 may extract a plurality of UA time-series windows 458, similar to the UA time-series windows 408 extracted from stored UA time-series data 402 described above. The UA time-series windows 458 may be input into trained UA detection model 430 at predefined inference intervals, in an overlapping fashion. At each predefined time interval, trained UA detection model 430 may output a UA time-series window 460. The UA time-series windows may then be aggregated at a window aggregator 462 to generate a continuous UA trace 464, that when plotted shows the UA, similar to plot 722 of FIG. 7. Thus, after training, trained UA detection model 430 may be able to detect and identify uterine contractions in raw ultrasound data acquired via a probe.

In some examples, the UA time-series windows 460 output by trained UA detection model 430 may be used as training data for a separate UA prediction model 470, which may predict a next uterine contraction for each UA time-series window 460. UA prediction model 470 is described in greater detail below in reference to FIG. 5.

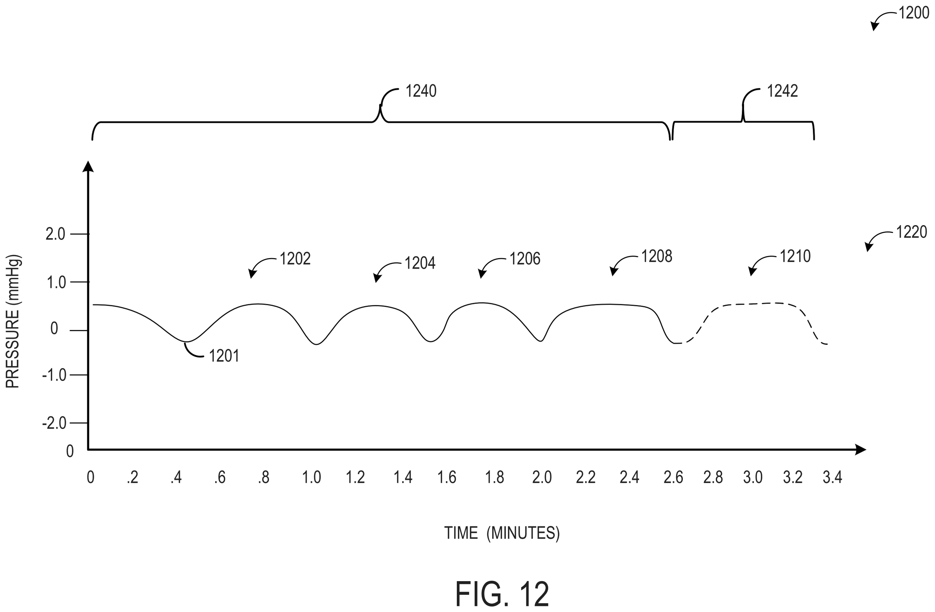

Referring now to FIG. 5, a method 500 is shown for training a UA detection model, such as UA detection model 420 of FIG. 4, according to an exemplary embodiment. The UA detection model may be trained in accordance with the UA detection model training system 400. In some embodiments, the UA detection model may be a deep neural network with a plurality of hidden layers. For example, the UA detection model may be a convolutional neural network (CNN), as described below in reference to FIG. 12. In other embodiments, the UA detection model may be a recurrent neural network (RNN), where some outputs of nodes of the RNN are used to affect subsequent inputs into the nodes. In still other embodiments, the UA detection model may be a different type of artificial neural network.

Method 500 and the other methods described herein may be executed by a processor of a UA detection system, such as UA detection system 302 of FIG. 3. Operations of method 500 may be stored in non-transitory memory of the UA detection system (e.g., in a training module such as the training module 310 of the UA detection system 302 of FIG. 3) and executed by a processor of the UA detection system (e.g., processor 304). The UA detection model may be trained on training data generated as described in reference to FIG. 4.

Method 500 begins at 502, where method 500 includes receiving UA time-series data of a plurality of subjects. The UA time-series data may include, for each subject of the plurality of subjects, a first dataset of physiological time-series data to be used as input into the UA detection model during training (e.g., input dataset 404), and a second dataset of physiological time-series data to be used as ground truth data for training the UA detection model (e.g., target dataset 405), where the first and second datasets have data acquired at the same time. That is, the first dataset and the second dataset may be acquired simultaneously by different data acquisition systems. For example, the first dataset may include ultrasound data acquired using an ultrasound probe during a patient visit, and the second dataset may include toco data acquired via a toco belt simultaneously with the ultrasound probe during the same patient visit. Alternatively, the second dataset may include EHG data acquired via an EHG device worn by the patient and acquired at the same time as the ultrasound data.

At 504, method 500 includes extracting data values of the first dataset into a first plurality of time-series windows of a predefined time (e.g., UA time-series windows 408 of FIG. 4). For example, the predefined time may be 10 seconds. The time-series windows may be created as described above in reference to FIG. 4.

The time-series windows may be generated in an overlapping fashion. For example, a first time-series window of 10 seconds may be created from the first dataset of the UA time-series data. The first time-series window may include 100 individual data values (e.g., measured pressures) starting with a first data value and ending with a 100^{th} data value of the first dataset. Each data value may be collected from a corresponding subject at the predetermined time interval. A second time-series window of 10 seconds may be created from the first dataset including 100 data values, starting with a second data value and ending with an 101^{st} data value of the UA time-series data, such that the second time-series window is offset from the first time-series window by one data value, and overlaps the first time-series window when plotted on a timeline. A third time-series window of 10 seconds may be created from the first dataset including 100 data values, starting with a third data value and ending with an 103^{th} data value of the first dataset, such that the third time-series window is offset from the second time-series window by one data value and from the first time-series window by two data values, and overlaps the first time-series window and the second time-series window when plotted on the timeline, and so on.

An example of the extraction and processing of the overlapping time-series windows during training is shown in FIGS. 9 and 10. Referring to FIG. 9, a time-series diagram 900 shows time-series UA data of a subject plotted on a line 902. A measured pressure detected in a reflected ultrasound signal of an ultrasound probe is represented on the Y axis, and time in seconds is represented on the X axis. A depicted portion of the time-series UA data includes a first uterine contraction 904. The time-series UA data may be collected continuously from the subject over a period of time. For example, the subject may be a patient, and the time-series UA data may be collected from the patient while an ultrasound scan is performed on the patient.

Individual UA data (e.g., pressure) values may be extracted at regular intervals from the time-series UA data, to generate a UA data array 908 that may be continuously updated over time. For example, a first exemplary data value 910 (e.g., 0.4 mmHg) corresponds to a beginning of first uterine contraction 904, and a second exemplary data value 912 (e.g., 0.3 mmHg) corresponds to an end of first uterine contraction 904. The extracted data values may then be further organized into a plurality of overlapping time-series windows, as shown in FIG. 10.

FIG. 10 shows a diagram 1000 depicting three overlapping time-series windows extracted from UA data array 908, including a first time-series window 1002, a second time-series window 1004, and a third time-series window 1006. The time-series windows 1002, 1004, and 1006 may be offset from each other by a predefined interval. In FIG. 10, the predefined interval is equal to the intervals at which the UA data values are extracted from the time-series data into array 908 of FIG. 9. In other embodiments, a predefined interval of a different length may be used. For example, the UA time-series data of FIG. 9 may be collected every 0.1 seconds, and time-series windows 1002, 1004, and 1006 may be offset from each other by 0.2 seconds, or 0.3 seconds, or a different amount of time.

At the passage of each predefined interval, a new time-series window may be created, as described above in reference to FIG. 4. The UA detection model may learn to perform sliding window inferences on the UA data included in the time-series windows over various inference intervals, where a single time-series window of UA data is input into the UA detection model at each inference interval.

For example, at a first inference interval, a first data value of UA data array 908 may be assigned to a first position of a first time-series window 1002. At a second inference interval, a second data value of UA data array 908 may be assigned to a second position of first time-series window 1002. Additionally, the second data value may be assigned to a first position of a second time-series window 1004. At a third inference interval, a third data value of UA data array 908 may be assigned to a third position of first time-series window 1002, a second position of second time-series window 1004, and a first position of a third time-series window 1006, and so on. In this way, a plurality of overlapping time-series windows of UA time-series data may be extracted from UA data array 908, where each overlapping time-series window is offset from a previous time-series window and a subsequent time-series window by the inference interval.

When the predefined number of data values of an overlapping time-series window has been obtained, meaning that a data value is included in each position of the relevant time-series window, the relevant time-series window may be stored for inputting into the UA detection model. A first dashed line 1010 shows a completion of first time-series window 1002. A second dashed line 1012 shows a completion of second time-series window 1004, and a third dashed line 1014 shows a completion of third time-series window 1006. When each time-series window includes the predefined number of data values, the time-series window may be stored. A timing of the overlapping time-series windows may be preserved, such that the overlapping time-series windows may be input into the UA detection model in order in a continuous fashion. After the passage of a subsequent inference interval, a next time-series window may be stored, and so on.

Returning to method 500, at 506, method 500 includes extracting data values of the second dataset into a second plurality of overlapping time-series windows of the predefined time. The second plurality of overlapping time-series windows may correspond temporally to the first plurality of overlapping time-series windows of the first dataset.

At 508, method 500 includes generating a plurality of training pairs, where each training pair of the plurality of training pairs includes a first array of data values corresponding to a time-series window of the first dataset, as input data, and a second array of data values corresponding to a corresponding time-series window of the second dataset as ground truth data. Once the training pairs have been created, the training pairs may be divided into a training dataset, a validation dataset, and a test dataset, as described above in reference to FIG. 4. An order of the overlapping time-series windows may be recorded and maintained for each dataset.

At 510, method 500 includes training the UA detection model on the training pairs. During training, the UA detection model may be configured to iteratively adjust one or more of a plurality of weights of the UA detection model in order to minimize a loss function, based on a difference between an output of the UA detection model for a given time-series window, and the corresponding ground truth target data for the relevant training pair. In one embodiment, the loss function is a Mean Absolute Error (MAE) loss function. It should be appreciated that the examples provided herein are for illustrative purposes, and other types of loss functions may be used without departing from the scope of this disclosure.

The difference (or loss), as determined by the loss function, may be back-propagated through the UA detection model to update the weights (and biases) of the hidden layers. In some embodiments, back propagation of the loss may occur in accordance with a gradient descent algorithm, wherein a gradient of the loss function (a first derivative, or approximation of the first derivative) is determined for each weight and bias of the UA detection model. Each weight (and bias) of the UA detection model is then updated by adding the negative of the product of the gradient determined (or approximated) for the weight (or bias) with a predetermined step size. Updating of the weights and biases may be repeated until the weights and biases of the UA detection model converge, or the rate of change of the weights and/or biases of the UA detection model for each iteration of weight adjustment are under a threshold.

At 512, in some examples, method 500 may include training a second, UA prediction model (e.g., UA prediction model 470) using the time-series windows output by the trained UA detection model. The UA prediction model may start as a copy or clone of the trained UA detection model. In such cases, the input data used to train the UA detection model may be input into the trained UA detection model to generate a set of overlapping output time-series windows indicating the UA in the input (e.g., ultrasound) data. The output overlapping time-series windows may then be used to generate training data for the UA prediction model.

To generate the training data, one training pair may be created for each overlapping time-series window output by the UA detection model. A first portion of a subsequent time-series window including a predefined number of values may be selected, where the first portion includes a uterine contraction. The first portion may be used as ground truth data for a preceding time-series window. Thus, the UA prediction model takes a series of overlapping time-series windows and learns to predict the predefined number of values having the next uterine contraction, based on the values of the preceding time-series window. A loss function may be used to backpropagate an error between the predicted values and the associated first portion, as described with respect to the UA detection model, until the UA prediction model is trained.

For example, each overlapping time-series window may include 100 values, with uterine contractions occurring roughly every 10 values. A first training pair is created with a first overlapping time-series window as input data, and the first 10 values of a second (next) overlapping time-series window as ground truth data; a second training pair is created with a second overlapping time-series window as input data, and the first 10 values of a third (next) overlapping time-series window as ground truth data; a third training pair is created with a third overlapping time-series window as input data, and the first 10 values of a fourth (next) overlapping time-series window as ground truth data; and so on. After the UA prediction model has been trained, the trained UA prediction model may take as input a continuous stream of UA time-series data output by the UA detection model, and output, for a current time or given time reference, a predicted next uterine contraction of a patient.

Turning now to FIG. 6, an exemplary method 600 is shown for deploying a trained UA detection model, such as trained UA detection model 430 of FIG. 4, to perform inferences on UA time-series data collected from a patient (e.g., a pregnant mother) of an ultrasound system (e.g., ultrasound system 100 of FIG. 1) to detect UA of the patient including a timing and duration of uterine contractions. In an embodiment, operations of method 600 may be stored in non-transitory memory of the UA detection system (e.g., in an inference module such as inference module 312) and executed by a processor of the UA detection system (e.g., processor 304).

Method 600 begins at 602, where method 600 includes acquiring ultrasound time-series data of the patient. In various embodiments, the ultrasound time-series data may be acquired via an ultrasound probe (e.g., probe 106) during a scan performed on the patient. The ultrasound time-series data may not be acquired by the same ultrasound system used to generate the training data. In other words, a first ultrasound system may be used to generate a first set of ultrasound time-series data used to train the UA detection model, and a second ultrasound system may be used to generate a second set of ultrasound time-series data for the performance of method 600.

At 604, method 600 includes extracting data values of the ultrasound time-series data into a plurality of overlapping time-series windows (e.g., UA time-series windows 408). The time-series windows may be created as described above in reference to FIG. 4.

At 606, method 600 includes inputting the plurality of overlapping time-series windows into the trained UA detection model, in order, at each predetermined time interval used to generate the overlapping time-series windows, to generate a respective plurality of output windows of UA time-series data (e.g., UA time-series windows 460). Then, at 608, method 600 includes aggregating the output windows of the UA time-series data to generate a first continuous UA trace (e.g., UA trace 464).

During the aggregation, the values of the output windows may be aggregated at each time interval. For example, a plurality of overlapping output windows may include predicted or estimated UA values for a given time t, and all the predicted or estimated UA values of the overlapping output windows may be averaged to generate an aggregate UA value for the time t. In other words, referring to FIG. 10, values in a first column 1050 corresponding to a first time may be averaged to determine a first aggregate value for the first time; values in a second column 1052 corresponding to a second time may be averaged to determine a second aggregate value for the second time (e.g., the first time plus the time increment); values in a third column 1054 corresponding to a third time may be averaged to determine a third aggregate value for the third time (e.g., the second time plus the time increment); and so on. By aggregating the UA values in this manner, the aggregate UA value calculated for time t is based on a plurality of estimations of the UA detection model made based on input data input at different times, which may increase a robustness of the output of the UA detection model.

At 610, method 600 includes generating and displaying a plot of the first continuous UA trace on a display screen (e.g., display device 334), where the plot may be viewed by an operator of the ultrasound system. The plot of the first continuous UA trace may be similar in appearance to toco plot 722 of FIG. 7. However, the plot may indicate the UA of the patient more precisely and accurately than a similar plot generated by a toco.

Method 600 also includes generating and displaying ultrasound physiological signals based on the ultrasound data input into the UA detection model. That is, the reflected ultrasound signal (e.g., the unprocessed ultrasound data) received from the probe may be used to generate the plot, and concurrently, the reflected ultrasound signal may be processed by the ultrasound system to generate physiological signals (e.g., of a fetus of the mother). In various examples, the plot may be displayed concurrently with the display of the ultrasound physiological signals. For example, the plot may be displayed on a first display device, and the physiological signals may be displayed on a second display device at the same time, or the plot may be displayed on the first display device along with or superimposed on the ultrasound physiological signals. In this way, an operator of the probe may view the physiological signals while simultaneously viewing the plot showing the uterine contractions of the mother.

At 612, method 600 optionally includes inputting the plurality of output windows into a trained UA prediction model (e.g., UA prediction model 470), and aggregating as described above, to generate a second continuous trace with a predicted uterine contraction. The second continuous trace may be similar to the first continuous trace, but may also include a predicted next (e.g., future) uterine contraction positioned at an end of the second continuous trace, which may be updated at each time interval. At 614, method 600 optionally includes displaying the second continuous trace on the display device, and method 600 ends.

FIG. 12 shows an exemplary graph 1200 including a second continuous trace 1201 that may be displayed on the display device in accordance with method 600. Note that FIG. 12 shows inverted UA activity. Trace 1201 includes a first portion 1240 including detected uterine contractions 1202, 1204, 1206, and 1208, which are detected by the UA detection model. First portion 1240 may correspond to a window of time-series data output by the UA detection model, which may be input into the UA prediction model. Trace 1201 includes a second portion 1242, which includes a predicted uterine contraction 1210, which may be predicted by the UA prediction model based partly on the data of first portion 1240 (and partly on other consecutive windows of time-series data output by the UA detection model that are aggregated to generate trace 1201). The display of trace 1201 may be updated in real time, where at each time increment, predicted uterine contraction 1210 may be replaced with a detected uterine contraction, and a new predicted uterine contraction of the UA prediction model may be displayed at an end 1220 of trace 1201.

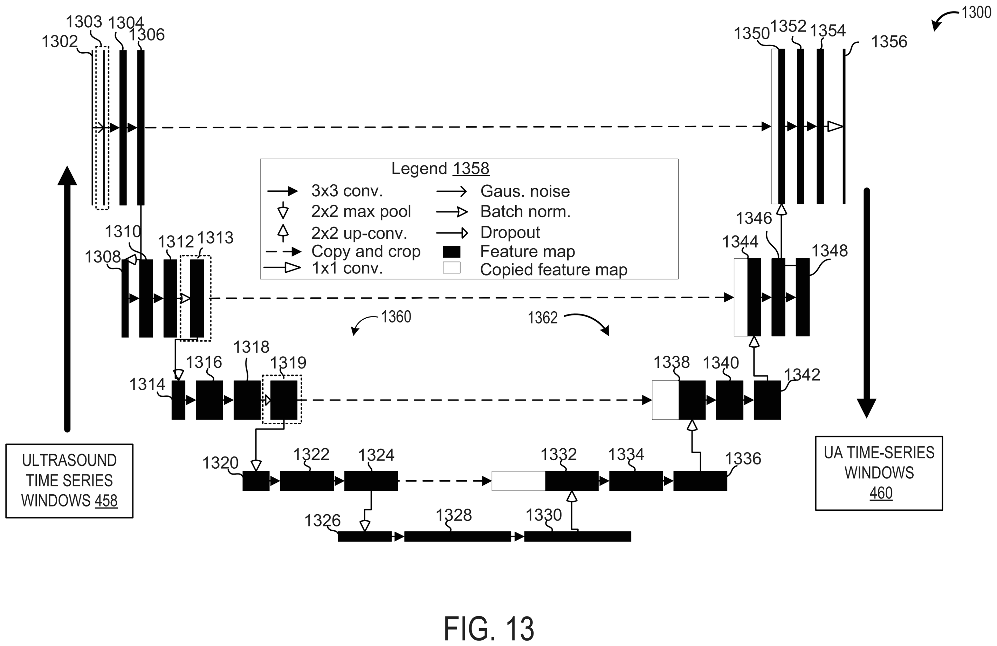

FIG 13 shows an architecture diagram of an exemplary CNN 1300, which may be used by the UA detection system, in an embodiment. FIG. 13 is provided by way of example, and it should be appreciated that in other embodiments, a different network architecture may be used without departing from the scope of this disclosure. CNN 1300 may take as input raw time-series data acquired via an ultrasound probe (probe 106), meaning, a receive signal reflected from anatomical structures within an abdomen of a pregnant mother prior to processing for physiological signals generation, and generate as output time-series data that when plotted creates a trace (UA trace 464) that represents uterine activity of the mother (e.g., uterine contractions). CNN 1300 represents a U-net architecture, which may be divided into an encoding portion (descending portion, elements 1302b-1330) and a decoding portion (ascending portion, elements 1332-1356). CNN architecture 1300 includes a series of mappings, from the set of ultrasound time-series windows 458 which may be received by an input layer 1302, through a plurality of feature maps, and finally to the set of UA time-series windows 460, which may be produced by an output layer 1356.

The various elements comprising CNN architecture 1300 are labeled in legend 1358. As indicated by legend 1358, CNN architecture 1300 includes a plurality of feature maps (and/or copied feature maps), wherein each feature map may receive input from a previous feature map, and may transform/map the received input to output to produce a next feature map. Each feature map may comprise a plurality of neurons, where in some embodiments, each neuron may receive input from a subset of neurons of a previous layer/feature map, and may compute a single output based on the received inputs, wherein the output may be propagated to a subset of the neurons in a next layer/feature map. A feature map may be described using temporal dimensions, such as frequency and duration (which may correspond to features of each sample of the input physiological signals) wherein the dimensions refer to the number of neurons comprising the feature map (e.g., the number of neurons along a length and the number of neurons along a width of a specified feature map).

In some embodiments, the neurons of the feature maps may compute an output by performing a dot product of received inputs using a set of learned weights (each set of learned weights may herein be referred to as a filter), wherein each received input has a corresponding learned weight, wherein the learned weight was learned during training of the CNN.

The transformations/mappings performed by each feature map are indicated by arrows, wherein each type of arrow corresponds to a distinct transformation, as indicated by legend 1358. Rightward pointing solid black arrows indicate 3×3 convolutions with stride of one, wherein output from a 3×3 grid of feature channels of an immediately preceding feature map are mapped to a single feature channel of a current feature map. Each 3×3 convolution may be followed by an activation function, wherein, in one embodiment, the activation function comprises a rectified linear unit (ReLU).

Downward pointing hollow arrows indicate 2×2 max pooling, wherein the max value from a 2×2 grid of feature channels is propagated from an immediately preceding feature map to a single feature channel of a current feature map, thereby resulting in an 8-fold reduction in spatial resolution of the immediately preceding feature map. In some examples, this pooling occurs for each feature independently.

Upward pointing hollow arrows indicate 2×2 up-convolutions, which comprise mapping output from a single feature channel of an immediately preceding feature map to a 2×2 grid of feature channels in a current feature map, thereby increasing the spatial resolution of the immediately preceding feature map 8-fold.

Rightward pointing dash-tailed arrows indicate copying and cropping of a feature map for concatenation with another, later occurring, feature map. Cropping enables the dimensions of the copied feature map to match the dimensions of the feature map with which the copied feature map is to be concatenated. It will be appreciated that when the size of the first feature map being copied and the size of the second feature map to be concatenated with the first feature map are equal, no cropping may be performed.

Rightward pointing arrows with hollow elongated triangular heads indicate a 1×1 convolution, in which each feature channel in an immediately preceding feature map is mapped to a single feature channel of a current feature map, or in other words, wherein a 1-to-1 mapping of feature channels between an immediately preceding feature map and a current feature map occurs.

Rightward pointing arrows with chevron heads indicate incorporation of Gaussian noise into a received input feature map.

Rightward pointing arrows with arcuate hollow heads indicate batch normalization operations, wherein a distribution of activations of an input feature map are normalized.

Rightward pointing arrows with a short hollow triangular head indicates a dropout operation, wherein random or pseudo-random dropout of input neurons (as well as their inputs and outputs) occurs during training.

In addition to the operations indicated by the arrows within legend 1358, CNN architecture 1300 includes solid filled rectangles corresponding to feature maps, wherein feature maps comprise a height (top to bottom length as shown in FIG. 13, corresponding to a y spatial dimension in an x-y plane), width (not shown in FIG. 13, assumed equal in magnitude to height, corresponding to an x spatial dimension in an x-y plane), and depth (a left-right length as shown in FIG. 13, corresponding to the number of features within each feature channel). Likewise, CNN architecture 1300 includes hollow (unfilled) rectangles, corresponding to copied and cropped feature maps, wherein copied feature maps comprise height (top to bottom length as shown in FIG. 13, corresponding to a y spatial dimension in an x-y plane), width (not shown in FIG. 13, assumed equal in magnitude to height, corresponding to an x spatial dimension in an x-y plane), and depth (a length from a left side to a right side as shown in FIG. 13, corresponding to the number of features within each feature channel).

Starting at input layer 1302, time-series data (e.g., ultrasound physiological signals) corresponding to uterine activity of a pregnant mother (ultrasound time-series windows 458) may be input into various layers. A first layer 1303 may be a wavelet decomposition transform layer that decomposes the input signal into primary frequency components (e.g., levels). First layer 1303 may be configured to have a same filter size and number of output channels to preserve a number of learning parameters. Only components of a specific frequency range may be preserved (e.g., level three to five), which may remove signal information that is irrelevant to output regression signal reconstruction.

The time-series data may be mapped to various sets of features in an encoder portion 1360 of CNN architecture 1300, followed by a decoder portion 1362 of CNN architecture 1300, ending at output layer 1356. The time-series data is acquired by scanning the abdomen of the mother using an ultrasound probe, in accordance with methods 500 and/or 600 of FIGS. 5 and 6, respectively.

In this way, CNN architecture 1300 may enable mapping of a first continuous stream of ultrasound time-series data to a second continuous stream of UA data that may be plotted to show a timing, duration, intensity, and/or other characteristics of uterine contractions of the mother. CNN architecture 1300 illustrates the feature map transformations which occur as the ultrasound time-series data is propagated through the neuron layers of the convolutional neural network, to produce the UA data.

The weights (and biases) of the convolutional layers in CNN 1300 are learned during training, as discussed in reference to FIGS. 5 and 6. Briefly, a loss function is defined to reflect the difference between the output data generated by CNN 1300 and corresponding ground-truth data acquired via a different sensing device, such as a toco, an EHG device, or a different device. The loss may be back-propagated through the layers of the neural network to update the weights (and biases) of the convolutional layers. CNN 1300 may be trained on a plurality of training pairs comprising overlapping windows of ultrasound time-series data, and corresponding ground truth UA time-series data acquired at a same time from a same subject, such that CNN 1300 learns to perform sliding window inferences to convert a first continuous stream of ultrasound time-series data to a second continuous stream of UA time-series data.

It will be appreciated that the current disclosure encompasses neural network architectures comprising one or more regularization layers, including batch normalization layers, dropout layers, Gaussian noise layers, and other regularization layers known in the art of machine learning which may be used during training to mitigate overfitting and increase training efficiency while reducing training duration. Regularization layers are used during CNN training and deactivated or removed during post training implementation of the CNN. These layers may be interspersed between the layers/feature maps shown in FIG. 13, or may replace one or more of the shown layers/feature maps.

It should be understood that the architecture and configuration of CNN 1300 shown in FIG. 13 is for illustration, not for limitation, and other appropriate neural networks may be used herein, such as ResNet, recurrent neural networks, General Regression Neural Network (GRNN), etc. Open source models such as Meta's Lag-Llama may also be used.

Thus, a solution to the problem of accurately recording and predicting uterine contractions while relying on a minimum number of sensing devices is provided. Specifically, a UA trace may be generated from ultrasound, EHG, or other time-series data acquired via a first device, that may be more accurate than a second trace generated by a second device, such as a toco. The UA trace may detect and/or predict uterine contractions, and indicate a timing, period, duration, intensity and/or other characteristics of the contractions based on the output of the first device. As a result, a reliance on the second device may be reduced. For example, via the methods disclosed herein, data acquired via an ultrasound probe that is already being used to generate physiological signals of a fetus and/or detect a heartbeat of the fetus during typical EFM may also be used to detect and predict uterine contractions, whereby an additional device for sensing the uterine contractions such as a toco or an EHG device may not be used. By eliminating a demand for the additional device, a cost of the EFM may be reduced. Further, the UA detection model may more accurately detect uterine contractions than the additional device. A second, UA prediction model may also be trained to predict a next uterine contraction from a set of previous uterine contractions based on an output of the trained UA detection model.

The technical effect of detecting and predicting UA from an ultrasound receive signal and/or EHG data, using a neural network model trained using ultrasound time-series data and/or EHG data as input data and time-series toco data as ground truth data, is that after training, the trained UA detection model may be used to detect a timing, period, duration, and intensity of uterine contractions in new patients with more accuracy than the toco, and without the use of an additional sensing device. Because the UA detection model is trained on a large training dataset spanning a wide variety of subjects of different ages, body types, races, etc., the UA detection model may learn to map the ultrasound data to UA time-series data based on concurrences between data acquired via different modalities, while ignoring the variance in individual patient data that causes inaccuracies in each modality alone. Because the trained UA detection model can be applied to ultrasound data already being recorded to capture a heartbeat of the fetus, a reliance on the toco sensor hardware for contraction detection may be reduced or completely eliminated. The ECG events occur on a time-scale of milliseconds, however, UA events occur on the time-scale of minutes. Therefore, as previously explained in FIG. 8, Ultrasound input signals scaled in time to the order of minutes can also be used to with the UA detection model to detect or predict UA activity using toco as the ground truth during training.

The disclosure also provides support for a method, comprising: acquiring ultrasound time-series data of an abdomen and/or a fetus of a pregnant mother, inputting the ultrasound time-series data into a first trained neural network model, generating a plot of a uterine activity (Ua) of the mother or the fetus from an output of the first trained neural network model, and displaying the plot on a display device. In a first example of the method, the ultrasound time-series data is acquired from a receive signal of an ultrasound probe prior to processing the ultrasound time-series data to generate ultrasound physiological signals. In a second example of the method, optionally including the first example, the ultrasound physiological signals are displayed on the display device concurrently with the plot. In a third example of the method, optionally including one or both of the first and second examples, the first trained neural network model is trained using stored ultrasound time-series data acquired from a plurality of subjects as input data, and stored time-series data of the mother's abdomen acquired from the same plurality of subjects at a same time as the stored ultrasound time-series data using a tocodynamometer as ground truth data. In a fourth example of the method, optionally including one or more or each of the first through third examples, the first trained neural network model is trained using stored time-series data of the mother's abdomen and/or fetus acquired from a plurality of subjects using an electrohysterogram (EHG) device as input data, and stored time-series data of the mother's abdomen and/or fetus acquired from the same plurality of subjects at a same time as the stored EHG time-series data using a tocodynamometer as ground truth data. In a fifth example of the method, optionally including one or more or each of the first through fourth examples, inputting the ultrasound time-series data into the first trained neural network model further comprises: generating a plurality of overlapping ultrasound time-series windows of the ultrasound time-series data at predefined increments of time, and inputting an overlapping ultrasound time-series window of the plurality of overlapping ultrasound time-series windows into the first trained neural network model at each increment of time. In a sixth example of the method, optionally including one or more or each of the first through fifth examples, generating a plot of the UA of the mother from the output of the first trained neural network model further comprises: receiving as an output of the first trained neural network model a plurality of overlapping windows of UA time-series data, each overlapping window of UA time-series data generated at the predefined increment of time, aggregating the plurality of overlapping windows of the UA time-series data by averaging values associated with a same time in each overlapping window, to create a continuous stream of UA time-series data, generating the plot of the UA from the continuous stream of UA time-series data. In a seventh example of the method, optionally including one or more or each of the first through sixth examples, the method further comprises: generating training data for a second trained neural network model from the plurality of overlapping windows of UA time-series data, the training data including training pairs comprising an overlapping window of UA time-series data as input data, and a portion of a subsequent overlapping window of UA time-series data as ground truth data, and using the training data, training the second trained neural network to predict a subsequent uterine contraction from a series of preceding uterine contractions. In a eighth example of the method, optionally including one or more or each of the first through seventh examples, the first trained neural network model comprises one of a convolutional neural network (CNN) and a recurrent neural network (RNN). In a ninth example of the method, optionally including one or more or each of the first through eighth examples, the first trained neural network model is a CNN, and a first layer of the CNN is a wavelet decomposition transform layer that decomposes an input signal of the CNN into primary frequency components.

The disclosure also provides support for a uterine activity (Ua) detection system, comprising: a display device, and a processor communicably coupled to the display device, and a non-transitory memory including instructions that when executed cause the processor to: during a first, training stage of a Ua detection model of the Ua detection system: generate a first plurality of overlapping windows of ultrasound time-series data acquired from a plurality of ultrasound scans performed on a plurality of fetuses and/or abdomens of pregnant mothers, generate a second plurality of overlapping windows of Ua time-series data acquired via a tocodynamometer (toco) from the pregnant mothers at a same time as the ultrasound time-series data, train the Ua detection model on training pairs including a time-series window of the first plurality of overlapping windows as input data and a time-series window of the second plurality of overlapping windows as ground truth data, and during a second, inference stage of the Ua detection model: receive ultrasound time-series data from an ultrasound probe placed over a fetus or a pregnant mother's abdomen, generate a third plurality of overlapping windows of the ultrasound time-series data, at predefined increments of time, input an overlapping window of the third plurality of overlapping windows into the trained Ua detection model, receive as outputs of the trained Ua detection model, at each predefined increment of time, a fourth plurality of overlapping windows of Ua time-series data, aggregate the fourth plurality of overlapping windows of Ua time-series data by averaging values associated with a same time in each overlapping window, to create a continuous stream of Ua time-series data, the continuous stream of Ua time-series data showing uterine contractions of the pregnant mother, and display a plot of the continuous stream of Ua time-series data on the display device. In a first example of the system, the ultrasound time-series data is acquired from a receive signal of an ultrasound probe prior to processing the ultrasound time-series data to generate an ultrasound physiological signals. In a second example of the system, optionally including the first example, further instructions are stored in the memory that when executed, cause the processor to process the ultrasound time-series data to generate an ultrasound physiological signals, and display the ultrasound physiological signals on the display device concurrently with the plot of the continuous stream of UA time-series data. In a third example of the system, optionally including one or both of the first and second examples, the UA detection model is trained using UA time-series data acquired from the pregnant mothers during the plurality of ultrasound scans via an electrohysterogram (EHG) device as ground truth data. In a fourth example of the system, optionally including one or more or each of the first through third examples, further instructions are stored in the memory that when executed, cause the processor to: generate training data for a UA prediction model from the plurality of overlapping windows of UA time-series data, the training data including training pairs comprising an overlapping window of UA time-series data as input data, and a portion of a subsequent overlapping window of UA time-series data as ground truth data, and using the training data, train the UA prediction model to predict a subsequent uterine contraction from a series of preceding uterine contractions. In a fifth example of the system, optionally including one or more or each of the first through fourth examples, the UA detection model comprises one of a convolutional neural network (CNN) and a recurrent neural network (RNN). In a sixth example of the system, optionally including one or more or each of the first through fifth examples, the UA detection model is a CNN, and a first layer of the CNN is a wavelet decomposition transform layer that decomposes an input signal of the CNN into primary frequency components.

The disclosure also provides support for a method, comprising: training a neural network model to output time-series data showing uterine activity (Ua) of a pregnant mother using, as input, ultrasound time-series data acquired via an ultrasound probe, wherein the neural network model is trained with a training data set including input data comprising ultrasound time-series data acquired from a plurality of pregnant mothers during a plurality of ultrasound scans, and ground truth data comprising tocodynamometer time-series data acquired from the plurality of pregnant mothers at a same time as the ultrasound time-series data. In a first example of the method, the method further comprises: inputting the ultrasound time-series data into the neural network model as a first plurality of overlapping windows of ultrasound data values, each overlapping window offset from a previous overlapping window by a predefined time increment, and receiving as an output of the neural network model a second plurality of overlapping windows of UA data values of the pregnant mother. In a second example of the method, optionally including the first example, the method further comprises: aggregating the second plurality of overlapping windows of UA data values by averaging values associated with a same time in each overlapping window, to create a continuous stream of UA time-series data, the continuous stream of UA time-series data showing uterine contractions of the pregnant mother.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (e.g., a material, element, structure, member, etc.) can be connected to or coupled to another obⱼect regardless of whether the one object is directly connected or coupled to the other object or whether there are one or more intervening objects between the one object and the other object. In addition, it should be understood that references to "one embodiment" or "an embodiment" of the present disclosure are not intended to be interpreted as excluding the existence of additional embodiments that also incorporate the recited features.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation and use may be made without departing from the principles and concepts set forth herein. Also, as used herein, the examples and embodiments, in all respects, are meant to be illustrative and should not be construed to be limiting in any manner.

## Claims

1. A method, comprising:
acquiring ultrasound time-series data of an abdomen and/or a fetus of a pregnant mother (602);
inputting the ultrasound time-series data into a first trained neural network model (606);
generating a plot of a uterine activity (UA) of the mother or the fetus from an output of the first trained neural network model (610); and
displaying the plot on a display device (610).

2. The method of claim 1, wherein the ultrasound time-series data is acquired from a receive signal of an ultrasound probe prior to processing the ultrasound time-series data to generate ultrasound physiological signals.

3. The method of claim 2, wherein the ultrasound physiological signals are displayed on the display device concurrently with the plot.

4. The method of claim 1, wherein the first trained neural network model is trained using stored ultrasound time-series data acquired from a plurality of subjects as input data, and stored time-series data of the mother's abdomen acquired from the same plurality of subjects at a same time as the stored ultrasound time-series data using a tocodynamometer as ground truth data.

5. The method of claim 1, wherein the first trained neural network model is trained using stored time-series data of the mother's abdomen and/or fetus acquired from a plurality of subjects using an electrohysterogram (EHG) device as input data, and stored time-series data of the mother's abdomen and/or fetus acquired from the same plurality of subjects at a same time as the stored EHG time-series data using a tocodynamometer as ground truth data.

6. The method of claim 1, wherein inputting the ultrasound time-series data into the first trained neural network model further comprises:
generating a plurality of overlapping ultrasound time-series windows of the ultrasound time-series data at predefined increments of time (604); and
inputting an overlapping ultrasound time-series window of the plurality of overlapping ultrasound time-series windows into the first trained neural network model at each increment of time (606).

7. The method of claim 6, wherein generating a plot of the UA of the mother from the output of the first trained neural network model further comprises:
receiving as an output of the first trained neural network model a plurality of overlapping windows of UA time-series data, each overlapping window of UA time-series data generated at the predefined increment of time;
aggregating the plurality of overlapping windows of the UA time-series data by averaging values associated with a same time in each overlapping window, to create a continuous stream of UA time-series data (608);
generating the plot of the UA from the continuous stream of UA time-series data (610).

8. The method of claim 7, further comprising:
generating training data for a second trained neural network model from the plurality of overlapping windows of UA time-series data, the training data including training pairs comprising an overlapping window of UA time-series data as input data, and a portion of a subsequent overlapping window of UA time-series data as ground truth data (508); and
using the training data, training the second trained neural network to predict a subsequent uterine contraction from a series of preceding uterine contractions (512).

9. The method of claim 1, wherein the first trained neural network model comprises one of a convolutional neural network (CNN) and a recurrent neural network (RNN).

10. The method of claim 9, wherein the first trained neural network model is a CNN, and a first layer of the CNN is a wavelet decomposition transform layer that decomposes an input signal of the CNN into primary frequency components.

11. A uterine activity (UA) detection system (302), comprising:
a display device (334); and
a processor (304) communicably coupled to the display device (334), and a non-transitory memory (306) including instructions that when executed cause the processor (304) to:
during a first, training stage of a UA detection model (420) of the UA detection system (302):
generate a first plurality of overlapping windows (408) of ultrasound time-series data (402) acquired from a plurality of ultrasound scans performed on a plurality of fetuses and/or abdomens of pregnant mothers;
generate a second plurality of overlapping windows (408) of UA time-series data (402) acquired via a tocodynamometer (toco) from the pregnant mothers at a same time as the ultrasound time-series data;
train the UA detection model (420) on training pairs (412) including a time-series window (408) of the first plurality of overlapping windows as input data (404) and a time-series window of the second plurality of overlapping windows as ground truth data (405); and
during a second, inference stage of the UA detection model (420):
receive ultrasound time-series data (456) from an ultrasound probe placed over a fetus or a pregnant mother's abdomen (452);
generate a third plurality of overlapping windows (458) of the ultrasound time-series data;
at predefined increments of time, input an overlapping window (458) of the third plurality of overlapping windows (458) into the trained UA detection model (430);
receive as outputs of the trained UA detection model (430), at each predefined increment of time, a fourth plurality of overlapping windows of UA time-series data (460);
aggregate the fourth plurality of overlapping windows of UA time-series data (460) by averaging values associated with a same time in each overlapping window, to create a continuous stream of UA time-series data (464), the continuous stream of UA time-series data (464) showing uterine contractions of the pregnant mother (452); and
display a plot of the continuous stream of UA time-series data on the display device.

12. The UA detection system (302) of claim 11, wherein the ultrasound time-series data (456) is acquired from a receive signal of an ultrasound probe prior to processing the ultrasound time-series data (456) to generate an ultrasound physiological signals.

13. The UA detection system (302) of claim 11, wherein further instructions are stored in the memory (306) that when executed, cause the processor (304) to process the ultrasound time-series data (456) to generate an ultrasound physiological signals (720), and display the ultrasound physiological signals (720) on the display device (334) concurrently with the plot (722) of the continuous stream of UA time-series data (464).

14. The UA detection system (302) of claim 11, wherein the UA detection model (420) is trained using UA time-series data (402) acquired from the pregnant mothers during the plurality of ultrasound scans via an electrohysterogram (EHG) device as ground truth data.

15. The UA detection system (302) of claim 11, wherein further instructions are stored in the memory (306) that when executed, cause the processor (304) to:
generate training data for a UA prediction model (420) from the plurality of overlapping windows (408) of UA time-series data (402), the training data including training pairs (412) comprising an overlapping window (408) of UA time-series data (402) as input data, and a portion of a subsequent overlapping window (408) of UA time-series data (402) as ground truth data (405); and
using the training data, train the UA prediction model (420) to predict a subsequent uterine contraction (1210) from a series of preceding uterine contractions (1202, 1204, 1206, 1208).
